(19) Europäisches Patentamt
European Patent Office
Office européen des brevets

(11) **EP 2 037 282 A2**

(12) **EUROPEAN PATENT APPLICATION**

(43) Date of publication:
**18.03.2009 Bulletin 2009/12**

(51) Int Cl.:
**G01N 35/00** (2006.01)

(21) Application number: **08252044.6**

(22) Date of filing: **13.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **15.06.2007 US 934649 P**
**20.06.2007 US 954252 P**

(71) Applicant: **ORTHO-CLINICAL DIAGNOSTICS, INC.**
**Rochester,**
**New York 14626-5101 (US)**

(72) Inventor: **Denton, Gary**
**Honeyoe Falls, NY 14472 (US)**

(74) Representative: **Goodfellow, Hugh Robin et al**
**Carpmaels & Ransford,**
**43-45 Bloomsbury Square**
**London**
**WC1A 2RA (GB)**

Remarks:
A request for correction for figure 7 has been filed pursuant to Rule 139 EPC. A decision on the request will be taken during the proceedings before the Examining Division (Guidelines for Examination in the EPO, A-V, 3.).

(54) **Clinical diagnostic analyzer performance estimator**

(57) The disclosed methods and devices relate to tools for on-site and customizable evaluation of the performance of clinical diagnostic analyzers or automation systems by executing a portable simulation program utilizing actual data from a site of interest. The portable simulation program consists of an Intelligent LIS preprocessor, implementation of a simulation model, and analytical and graphical means to determine and present the performance metrics. This permits a realistic evaluation of the clinical diagnostic analyzers before installing the equipment, thus aiding in making decisions including a decision to buy or appropriately configure clinical diagnostic analyzer. The analysis also allows customers to modify the laboratory setup, improve delivery schedules, with better sample scheduling and storage, and in adding equipment.

FIGURE 1A

**(Cont. next page)**

Start

Preprocessing

110

Specifying the Clinical Diagnostic
Analyzer

120

Selecting Parameters for the
Simulation

130

Providing Metrics on the Analyzer
Performance

140

End

**FIGURE 1B**

**Description**

**FIELD OF THE INVENTION**

The disclosed methods and devices relate to tools for on-site and customizable evaluation of the performance of clinical diagnostic analyzers or automation systems by executing a portable simulation program utilizing actual data from a site of interest.

**BACKGROUND**

Estimating the expected performance of clinical laboratory testing and diagnostic systems requires detailed information about the expected workload for accurate capacity planning and performance modeling making such modeling expensive. Clinical laboratory testing and diagnostic systems include components and subsystems that are subject to Food and Drug Administration of the United States or similar regulatory agency in other jurisdictions. Most components cannot be modified to meet regulatory compliance. However, such components can be combined to provide customization and, naturally, this adds to the complexity in carrying out a simulation. Further, highly predictable performance of these systems is required because accuracy and time taken to provide test results may be critical. Thus, investment decisions lead to overcapacity or undercapacity due to inaccurate information about the true needs. Typical assays conducted by such systems cover the spectrum from immunological tests to testing for electrolyte levels, and for metabolites and drugs-including legal and contraband substances, which also contribute to the complexity of evaluating a system for performance in a particular context.

Laboratories differ significantly in their use of laboratory testing and diagnostic systems. Physicians typically need fast turnaround times for results to allow them to effectively monitor patients under care. For instance, patients with kidney problems or trauma need to be evaluated and monitored to properly manage their electrolyte levels. Further, not all clinical laboratories support all tests or even need the same mix of tests making local considerations important in evaluating analyzers.

For various reasons, when evaluating clinical diagnostic analyzers, a crude measure-the peak-processing rate-is used to estimate the time and capacity required to process an expected sample workload. This is not a satisfactory solution because it treats other factors as being irrelevant.

No satisfactory cost effective simulation packages are available for evaluating a clinical diagnostic analyzer of interest. For instance, United States Patent No. 7,076,474 describes a method to simulate a new business process using historical execution data. This method requires the new business process to be represented by a directed graph with various node types such as start nodes, arc nodes, route nodes, work nodes and termination nodes, which require execution data, from which simulation parameters are derived. Such a representation is unnecessarily complex for evaluating clinical diagnostic analyzers.

United States Patent Publication No. 2006/0031112 discloses a 'simulation' that recommends equipment based on similarity to past experiences in providing equipment to other clients. Thus, the method is limited by the need for a database of similarly situated clients and the assumption that the products are improved insignificantly in the interim to allow meaningful comparisons to be made to the database records. More importantly, the method ignores the specific needs or the specific systems being considered.

United States Patent Publication No. 2007/0078692 discloses a simulation method that uses a hierarchical inverted tree structure to model the process being simulated. A clinical diagnostic analyzer includes loops, such as those formed when samples are reflexed for retesting, and that preclude modeling them as inverted hierarchical trees.

United States Patent No. 6,768,968 discloses a strategy for estimating the performance of a computer system. While a clinical diagnostic analyzer includes processing power, it is not quite like a computer system in view of its highly specialized properties. The computer is integrated into electrical and mechanical parts for accurately testing biological materials, which is the primary purpose of the device.

Nevertheless, purchasing decisions attempt to take into account even rough estimates of factors such as the highest patient-reportable result efficiency (First Pass Yield), real-time access to information for proactive diagnosis and remote repair, self-monitoring, intelligent system fault recovery, minimal maintenance, no reagent preparation and calibration stability. First Pass Yield helps reduce non-value-added tasks, which also improves morale and staff retention. Further, it is desirable to have real-time access to information for proactive diagnosis and remote repair to improve uptime and increase productivity. Such systems should also be self-monitoring, with intelligent system fault recovery, few maintenance requirements, and nominal or reduced reagent preparation while displaying calibration stability over a long time period resulting in fewer intervention steps to deliver true walk-away operation. During walk-away operation, the operator may have respite or carry out alternative tasks.

These features, although valued, are not captured by gross statistics of maximum throughput. Further, back of the envelope estimates do not accurately capture the savings or costs in adopting a particular system. These systems cost in the range of three hundred thousand dollars or more making deployment decisions rather important.

## SUMMARY

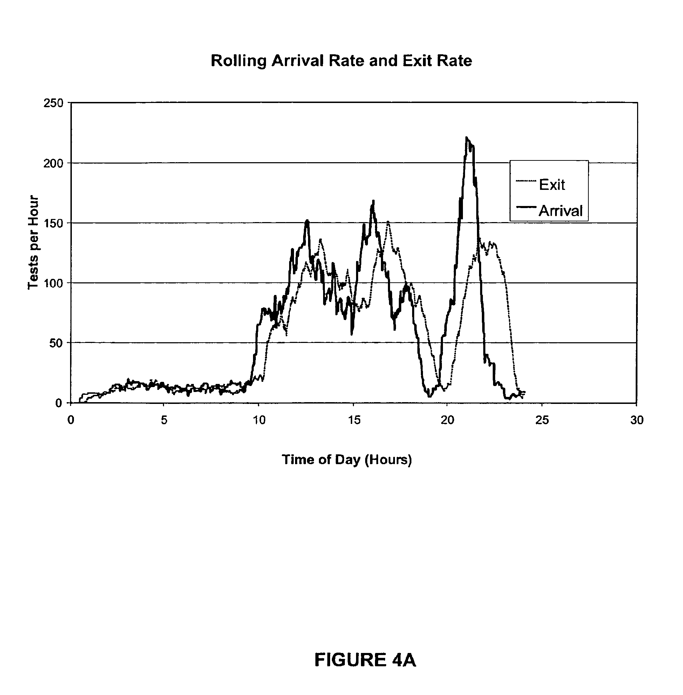

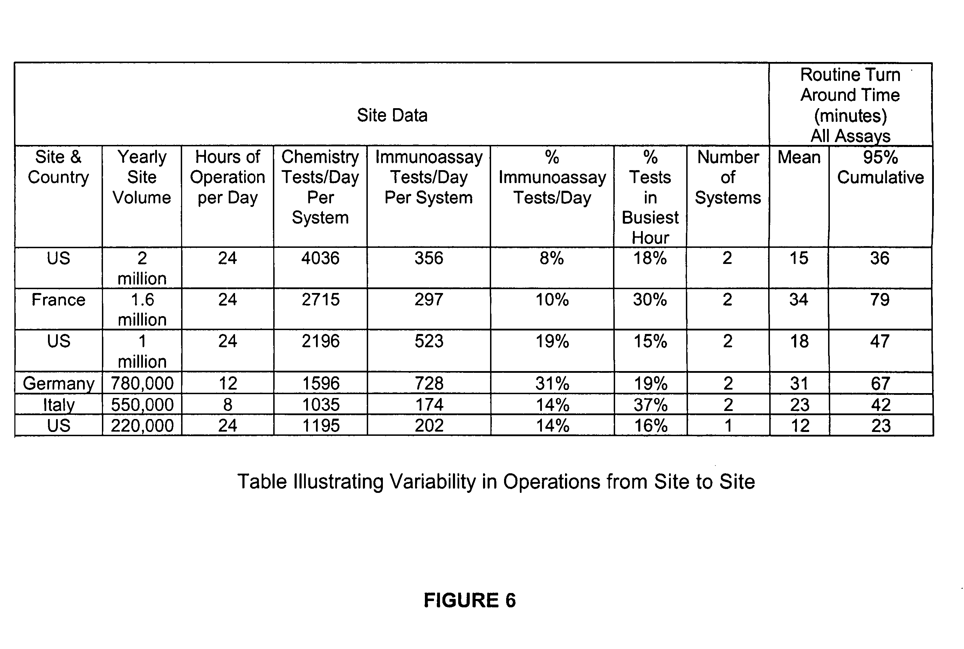

Applicants recognized the need for evaluation of clinical diagnostic analyzers performance based on local needs. Figure 6 and the illustrative graphs for two different sites shown in Figures 4A-B demonstrate the influence of multiple factors on the performance of a clinical analyzer in a particular place as well the considerable difference in the demands made on the instrument in different contexts.

Applicants recognized that local needs are preferably approximated by historical or expected test volumes and test type distributions to better estimate the cost of ownership. Applicants have further devised a portable and computationally undemanding tool for practical and quick simulation of just such an evaluation-before the actual system is deployed. This makes it possible to customize design and other details based on tool performance.

A portable simulation method and system is disclosed for evaluating laboratory testing and diagnostic systems, such as clinical diagnostic analyzers. The disclosed evaluation methods and devices are efficient and cost effective because their evaluation reflects actual expected usage and provides metrics tailored to aid in addressing cost or management issues. The disclosed method and system rely on a simulation, which allows estimation of the performance of a clinical diagnostic analyzer or automation system at each specific customer site before actual deployment. It consists of an Intelligent LIS pre-processor, a simulation model, and analytical and graphical means. Using the disclosed method and systems, the performance of a clinical analyzer or automation system can be estimated with greater precision before the buying decision is made or equipment installed.

A sales person may use the disclosed method and system using little more than a laptop computer or even a portable memory device encoded with computer executable instructions. With the aid of the disclosed method and system, a potential customer can get a detailed picture of various available options without making extensive investments of time and money. Further, any data provided by the customer continues to be secure in compliance with patient privacy requirements.

The results of the simulation include a Maximum Turn Around Time, an Average Turn Around Time, a 95% Turn Around Time, a Maximum Throughput, Consumable Usage, a Walk-Away Time, and a Downtime. These results allow customers to improve their estimates of resource and capital needs, modify the laboratory setup, adjust delivery schedules, or adding equipment to better meet their needs based on predictable financials while eliminating many of the hidden costs, maintenance and productivity issues. Some preferred embodiments are described below with the aid of illustrative figures, which are briefly described next.

## BRIEF DESCRIPTION OF THE DRAWINGS

FIGS. 1A-B outline methods for carrying out a preferred simulation implementation.

FIGS. 2A-B illustrates some salient features in a preferred preprocessor.

FIG. 3 shows a flow chart underlying a preferred simulation implementation.

FIGS. 4A-B show output graphs showing the turnaround time and the input and output of clinical diagnostic analyzers at different locations.

FIG. 5 shows an illustrative graph of cumulative and % Turn Around Times in a preferred simulation implementation.

FIG. 6 shows a Table summarizing the differences in local conditions and use of clinical diagnostic analyzers at different locations requiring prediction of the configuration and type of clinical diagnostic analyzer by a preferred simulation implementation.

FIG. 7 illustrates the flow of samples through configuration comprising different types of clinical diagnostic analyzer in accordance with rules for controlling the flow of samples and tests.

## DETAILED DESCRIPTION OF THE PREFERRED EMBODIMENT

Deployment and design decisions relating to clinical diagnostic analyzers are likely to benefit from realistic estimates. For instance, the local conditions may strongly influence the peak throughput for an instrument being considered. The expected frequency and distribution of particular tests is often ignored in evaluating systems because taking them into account requires processing voluminous data. Taking such details into account is likely to improve the evaluation of candidate clinical diagnostic systems. The table in Figure 6 demonstrates the influence of multiple factors on the performance of a clinical analyzer in a particular place. This variability is also illustrated by the two example graphs for two different sites shown in Figures 4A-B.

Although accurate and more realistic estimates aid in configuring and deploying laboratory testing and diagnostic systems, such estimates are expensive and difficult to obtain when reviewing available candidate clinical laboratory testing and diagnostic systems. This difficulty in providing realistic projections of expected use of laboratory testing and diagnostic systems is overcome by this disclosure. Accordingly, now it is possible to deliver predictable financials by eliminating hidden costs, maintenance and productivity issues associated with alternative implementations- provided they are in a timely and cost effective manner. Such estimates are useful in selecting the appropriate configuration and capacity for the clinical diagnostic analyzers or automation systems at each specific customer site. Such estimates allow efficient and effective delivery of healthcare.

Some of the illustrative tests offered on clinical diagnostic analyzers are listed in the following ASSAY TYPE TABLE.

|  | | ASSAY NAME | ASSAY DESCRIPTION |
|---|---|---|---|
| | 1 | T4 | T4 , Total T4, Thyroxine |
| | 2 | T3Up | T3 Uptake, T3U |
| | 3 | CA153 | CA 15-3 |
| | 4 | Myo | Myoglobin |
| | 5 | HAVab | HAV Antibody |
| | 6 | RubG | Rubella IgG |
| | 7 | ToxoG | Toxoplasma IgG |
| | 8 | CMVG | CMV IgG |
| | 9 | FeT | Ferritin, Iron/Tissue |
| | 10 | PSAT | Prostate Specific Antig/Total, PSA |
| | 11 | T3T | T3 Total |
| | 12 | hCGF | HCG/Free, HCG-CTP |
| | 13 | HCVab | HCV Antibody |
| | 14 | T4F | T4, Free |
| | 15 | T3F | T3, Free |
| | 16 | Estr | Estradiol |
| | 17 | Prog | Progesterone |
| | 18 | TestT | Testosterone, Total |
| | 19 | NTelo | N-Telopeptide, NTx, Osteopor |
| | 20 | Cort | Cortisol |
| | 21 | CA125 | CA 125 |
| | 22 | CEA | CEA, Carcinoembryonic Antigen |
| | 23 | Prol | Prolactin |
| | 24 | FSH | Follicle Stimulating Hormone, FSH |
| | 25 | CKMB | Creatinine Kinase - MB, CKMB, MAss CKMB-Isoenzymes |
| | 26 | LH | LH, Leutinizing Hormone |
| | 27 | Trop | Troponin |
| | 28 | T4 | T4 , Total T4, Thyroxine |

(continued)

|  | ASSAY NAME | ASSAY DESCRIPTION |
|---|---|---|
| 29 | T3Up | T3 Uptake, T3U |
| 30 |  |  |
| 31 | TSH | TSH, 3rd Gen, High Sensitivity, Thyrotropin |
| 32 | HBSag | HBs Antigen |
| 33 | HIV | HIV (PMA) HIV 1/2 Antibody |
| 34 | Hbeag | HBe Antigen |
| 35 | Hbeab | HBe Antibody |
| 36 | HBSab | HBs Antibody |
| 37 | Fola | Folate, Folic Acid |
| 38 | B12 | Vitamin B12, Cobalamin |
| 39 | HBcMab | HBc Antibody IgM |
| 40 | HAVMag | HAV Antibody IgM |
| 41 | RubM | Rubella IgM |
| 42 | ToxoM | Toxoplasma IgM |
| 43 | CMVM | CMV IgM |
| 44 | hCGQ1 | HCG/Qualitative, bHCG |
| 45 | hCGQn | HCG/Quantitative, bHCG, Total bHCG |
| 46 | AFP | AFP, Alpha Fetoprotein |
| 47 | AIAT | AAT, A1AT, Alpha-1 antitrypsin, al-protease inhibitor |
| 48 | Amph | Amphetamines, Methamphetamine |
| 49 | ApoA1 | Apo A1, Apolipoprotein A1 |
| 50 | ApoB | Apo B, Apolipoprotein B |
| 51 | ASO | ASO, Antistreptolysin |
| 52 | Barb | Barbiturates, Seconal |
| 53 | Bnzo | Benzodiazepine, Halcion |
| 54 | C3 | C3, Complement C3 |
| 55 | C4 | C4, Complement C4 |
| 56 | Caf | Caffeine |
| 57 | ChlL | Cholesterol, LDL |
| 58 | Coca | Cocaine |
| 59 | CRPHS | CRPhs, C-Reactive Protein (high sensitity) |
| 60 | Cycl | Cyclosporin |
| 61 | DDim | D-Dimer, Fibrin degradation fragment |
| 62 | Digit | Digitoxin |
| 63 | FeBT | Iron Binding Capacity/ Total, TIBC |
| 64 | FeBU | Iron Binding Capacity/Unsaturated UIBC, (Calculated) |
| 65 | FK506 | FK506, Tacrolimus |
| 66 | Gent | Gentamicin |

(continued)

|  | ASSAY NAME | ASSAY DESCRIPTION |
|---|---|---|
| 67 | Hapt | Haptoglobin |
| 68 | HbA1C | Hemoglobin/Glycated, A1C |
| 69 | Hcyst | Homocysteine |
| 70 | IgA | IgA |
| 71 | IgG | IgG, Gamma Globulin |
| 72 | IgM | IgM |
| 73 | Lipo | Lipoprotein, Lipoprotein A |
| 74 | Mcalb | Microalbumin |
| 75 | Meth | Methadone |
| 76 | NAPA | procainamide/N-propionyl, NAPA |
| 77 | Opia | Opiates |
| 78 | PCP | Phencyclidine, PCP |
| 79 | Preal | Prealbumin |
| 80 | Procai | Procainamide |
| 81 | Quin | Quinidine |
| 82 | RF | Rheumatoid Factor |
| 83 | Siro | Sirolimus, Rapamune |
| 84 | THC | Cannabinoids, THC |
| 85 | Tobra | Tobramycin |
| 86 | Trans | Transferrin |
| 87 | Tricy | Tricyclic Antidepressants |
| 88 | Valp | Valproic Acid, Depakote |
| 89 | Vanco | Vancomycin |
| 90 | Acet | Acetamimophen, Tylenol |
| 91 | Alb | Albumin |
| 92 | ALP | Alkaline Phosphatase |
| 93 | ALT | ALT, SGPT, GPT, Alanine Transaminase |
| 94 | Amy | Amylase |
| 95 | AST | SGOT, AST, Aspartate Transaminase, Transferance |
| 96 | BiliD | Bilirubin/Direct, Conjugated |
| 97 | BiliI | Bilirubin/Indirect, Uncongugated |
| 98 | BiliT | Bilirubin/Total |
| 99 | BUN | BUN, Urea Nitrogen, URR |
| 100 | Ca | Calcium |
| 101 | Carb | Carbamazepine, Tegretol |
| 102 | Chl | Cholesterol, Total |
| 103 | ChlH | Cholesterol, HDL |
| 104 | Choli | Cholinesterase |

(continued)

|  |  | ASSAY NAME | ASSAY DESCRIPTION |
|---|---|---|---|
|  | 105 | CK | Creatinine Kinase, CK, CPK, CKNac Total |
|  | 106 | Cl | Chloride, Cl |
|  | 107 | CO2 | C02, Carbon Dioxide |
|  | 108 | Crea | Creatinine |
|  | 109 | CRP | CRP, C-Reactive Protein |
|  | 110 | Dig | Digoxin, Digitalis |
|  | 111 | Dil | Phenytoin , Dilantin |
|  | 112 | FeS | Iron/Serum |
|  | 113 | GGT | GGT, Gamma Glutamyl Transpeptidase |
|  | 114 | Glu | Glucose |
|  | 115 | K | Potassium |
|  | 116 | LA | Lactate, Lactic Acid |
|  | 117 | LDHIs | Lactate Dehydrogenase, LD, Lactic dehydrogenase, LDH, and LDH isoenzymes |
|  | 118 | Li | Lithium |
|  | 119 | Lip | Lipase |
|  | 120 | Mg | Magnesium, Mg |
|  | 121 | Na | Sodium |
|  | 122 | NH3 | Ammonia |
|  | 123 | OH | Alcohol/Quantitative |
|  | 124 | OHQ1 | Alcohol, Qualitative |
|  | 125 | P | Phosphorus |
|  | 126 | PAP | Acid Phosphtase, PAP |
|  | 127 | Pheno | Phenobarbital, Barbita, Luminal, Solfoton |
|  | 128 | Prot | Protein, Total Proteins, CSF Protein, Urine Protein |
|  | 129 | Salic | Salicylate, Aspirin |
|  | 130 | Theo | Theophylline |
|  | 131 | Trig | Triglyceride |
|  | 132 | Uric | Uric Acid |
|  | 133 | Hb | Hb |
|  | 134 | A1C | A1C |
|  | 135 | BiliN | Bilirubin/Direct, Conjugated |
|  | 136 | Propx | Propoxiphine, Darvon |

The supporting role of clinical diagnostic analyzers in providing a fast turnaround is an important factor in delivering the required level of healthcare. As a result, it is desirable to estimate processing times for relatively urgent samples (the STAT samples) separately from standard samples (the Routine samples) along with an overall combination of the total sample processing.

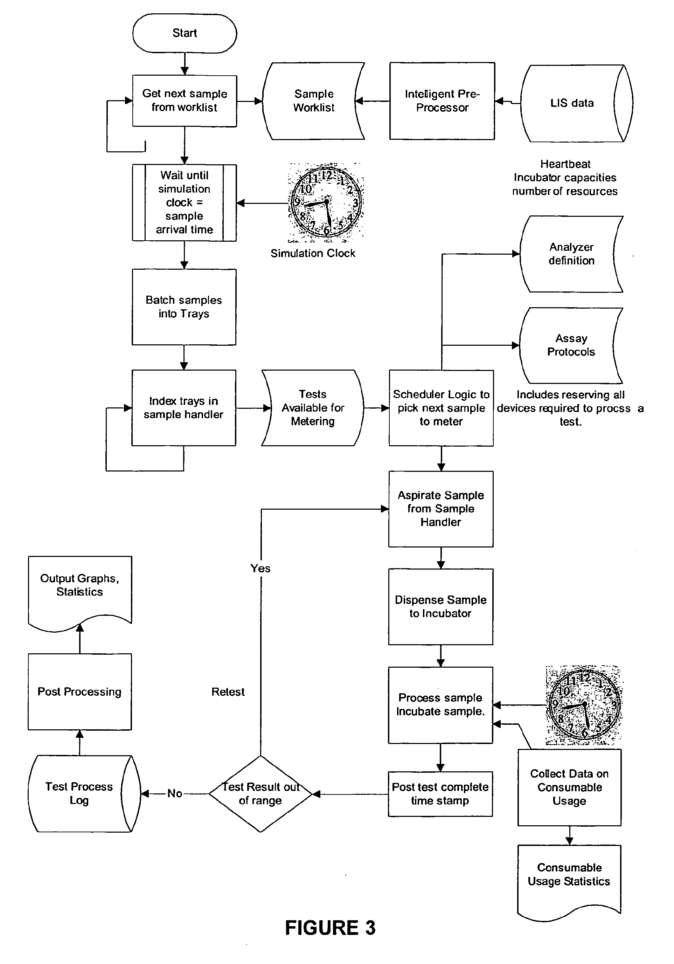

A clinical laboratory testing and diagnostic system typically includes a Scheduler, which controls and specifies operations in the analyzer. The Scheduler ensures that samples are accepted from an input queue as resources are reserved for the various expected tests relevant to a particular sample. Unless the required resources are available, a sample continues to be in the input queue. Samples are further batched into trays (or slots) as is shown in Figure 3. In a preferred analyzer model, the sample is aspirated and then sub-samples are taken from this aspirated volume for various tests. The operation of the Scheduler together with the types of tests, for instance the tests listed in the ASSAY TYPE TABLE above, supported by the analyzer provide a reasonably accurate description of an analyzer under consideration.

Typically such details are not only not available, they are cumbersome to evaluate because of the large number of combinations that reflect different outcomes. As a result, typically parameters such as turnaround time limits are provided to aid in comparing or evaluating the performance of analyzers. Applicants recognized that deploying laboratory testing and diagnostic systems benefits from considering more than just fast turnaround time limits. For instance, one study, summarized in the table presented in Figure 6, of such instruments revealed that clinical diagnostic analyzers handled yearly site volumes of as few as 220 thousand tests to as many as two million tests on each system. Some sites operated the systems round the clock (24 hours) while others operated for as few as eight (8) hours in a day. Chemistry assays, for instance to measure electrolytes like Sodium, Potassium, Carbonate and the like, performed on a single system ranged from about a thousand to more than four thousand in a day. Similarly, for immunoassays, from less than two hundred assays in some settings to more than five hundred assays were carried out on a single system in a day. The turnaround times for the assay results, the time taken from inputting the sample to receiving the output results, ranged from about twenty three (23) minutes to more than an hour while overall throughput and cost or ownership issues were also important factors.

Significant metrics for evaluating a clinical laboratory testing and diagnostic system include a Maximum Turn Around Time, which is the longest time period from sample arrival to reported test result in a set of samples; the Average Turn Around Time, which is the average time from sample arrival to reported test result; the 95% Turn Around Time (an example of a % Turn Around Time), which is the time under which 95% of the tests will reported; Maximum Throughput, which is the maximum number of tests per hour processed by the system and is the peak value of the sample exit rate in a graph of sample exit rate against time; Downtime, for which the analyzer is not available due to for instance, the need to maintain or calibrate the machine; Walk-Away Time, during which unattended operation is possible; and Arrival and Exit Rate, which may be presented in the form of a graph showing curves representing the arrival rate of test requests into the lab and the rate at which test results are posted by the analyzer (the result rate). These measures may be periodically updated in the form of rolling averages. Further, these metrics may be reported separately for Routine and STAT samples.

A preferred embodiment uses an Intelligent LIS preprocessor, a simulation model and an analytical and graphical means for providing the estimated performance metrics to a customer prior to the decision to purchase. The simulation is preferably based on data provided by the customer for a representative simulation. Such a simulation-based tool allows improved turn-around times, delivery schedules and rapid identification of desirable modifications to the laboratory setup.

The preferred embodiment comprises a plurality of components for computerized processing such that the components cooperate to implement the disclosed methods. The components in the system may be hardware, which may include an output device (e.g. a display device such as a screen, monitor or television, or a loudspeaker or telephone), a workstation, an input device (e.g., a keyboard, numerical keypad, dial, touch screen, touch pad, pointing device such as a mouse, microphone or telephone), or software (typically for configuring the hardware), or preferably a combination of hardware and software.

An exemplary system for implementing the invention comprises two or more components cooperating to implement the methods of the invention in a suitable computing environment, e.g., in the general context of computer-executable instructions. Generally, computer-executable instructions may be organized in the form of program modules, programs, objects, components, data structures and the like.

An exemplary system for implementing the invention includes a suitably configured general purpose computing device. The invention may be implemented using a wide variety of such devices including personal computers, hand-held devices, multiprocessor systems, microprocessor based or programmable consumer electronics, network PCs, minicomputers, and in the form of instructions stored on a portable storage device or media and the like including local or remote memory storage devices. The computing environment preferably includes a hard disk drive for reading from and writing to a hard disk, a magnetic disk drive for reading from or writing to a removable magnetic disk, or an optical disk drive for reading from or writing to a removable optical disk such as a CD ROM or other optical media. It will be appreciated by those skilled in the art that computer readable media which can store data accessible to a computer, such as a removable magnetic disk, and a removable optical disk, magnetic cassettes, flash memory cards, digital video disks, Bernoulli cartridges, random access memories, read only memories, and the like may also be used in the exemplary operating environment. Thus, the computing environment may include computer readable media such as volatile or nonvolatile, removable or non-removable media implemented in any technology or method for information storage such as computer instructions, data structures, program modules and the like. Computer storage media includes, but is not limited to, RAM, ROM, EEPROM, flash memory, or other memory technology, CD-ROM, CD-RW disks, Digital Versatile Disks ("DVD") etc. that can be used to store and access the information. Communication media typically includes computer readable instructions, data structures, program modules or data in a modulated data signal such as a carrier wave. These devices are often accessed by the processing unit through an interface, such as a serial port interface that is coupled to the system bus. Increasingly, such devices are being connected by the next generation of interfaces, such as a universal serial bus (USB) with a root hub/Host, and to which other hubs and devices may be connected. Other interfaces that may be used include parallel ports, game ports, and the FireWire, i.e., the IEEE 1394 specification.

The computing environment may be networked using logical connections to one or more databases and remote computers. The logical connections underlying the computing environment may include a local area network (LAN) and a wide area network (WAN) with wired or wireless links. Such networking environments are commonplace in offices, enterprise-wide computer networks, intranets and the Internet with client-server or peer-to-peer networking protocols. USB provides another way to connect to a network by either using a communication link via a wireless link, a modem, an ISDN connection and the like, or even a hub that can be connected to two computers. The network connections shown are exemplary and do not exclude other means of establishing a communications links.

In one preferred embodiment, these components comprise a personal computer, such as an IBM PC compatible or another computing platform. The simulation software is preferably coded using JAVA programming language, which allows it to be executed by a large number of computing platforms. Notably, in an actual clinical diagnostic analyzer a component like the Scheduler may be coded using a programming language like C++. The same scheduler performance may be simulated using modules coded in JAVA. It should also be noted that these software implementation features are not intended to limit the scope of the disclosure.

In a preferred embodiment the input data used for simulation is actual data collected over a period of about a day at the site of interest. Alternatively, the customer or party reviewing the performance of one or more clinical diagnostic analyzer configurations may select or approve the data. It should also be noted that in the following description, the term 'sample' actually denotes a pseudo-sample constructed for the purpose of carrying out the simulation rather than being subjected to an actual test. The analyzer definition utilized preferably includes the scheduling details used by an analyzer of interest and the time estimates for various tests and steps carried out.

In a preferred embodiment the Test arrival rate is updated at one-minute intervals. The computation logic may be represented as:

If (time now - time simulation start) < 1 hour

$$TestArrivalRate := \frac{NumberofTestRequests}{Timenow - StartTime} \frac{tests}{hour}$$

Else

$$TestArrivalRate := \frac{NumberofTestRequestsinLastHour}{1} \frac{tests}{hour}$$

Similarly, the Results Exit rate is also updated at one-minute intervals:

If (time now - time simulation start) < 1 hour

$$TestResultRate := \frac{NumberofTestResults}{Timenow - StartTime} \frac{tests}{hour}$$

Else

$$TestResultRate := \frac{NumberofTestResultsinLastHour}{1} \frac{tests}{hour}$$

The test arrival rate (the leading curve) and the results exit rate (the lagging curve) are shown in Figures 4A-B for two different locations. The x-axis of the graph is the time of day. The time gap between the leading curve and the lagging curve is the Turn Around Time. The difference between a higher leading curve peak and the corresponding lagging curve peak indicates an arrival rate that may be higher than the analyzer's maximum throughput. Such a difference does not disqualify an analyzer from being suitable. If the Turn Around Time gap does not exceed a specified maximum and the total workload is completed within a specified time, the analyzer may perform satisfactorily at the site of interest. When the leading curve maximum exceeds the corresponding lagging curve maximum, then samples will back up behind the analyzer during this period of the day. The laboratory also can use this curve to determine if the sample arrival schedule needs to be changed.

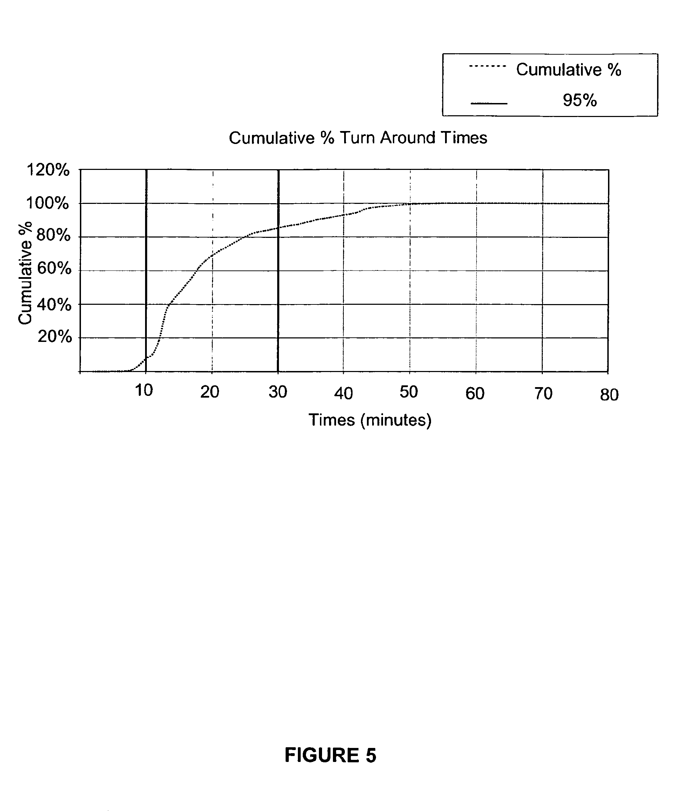

An example plot of Cumulative % Turn Around Times is shown in Figure 5. In Figure 5, the graph shows data collected at one-minute intervals. For each minute on the x-axis, the number of samples that have Turn Around Times equal to or less than that minute is totaled and divided by the total number of tests. The resulting percentage is plotted on the y-axis corresponding to the x-axis turn around time.

The flat line represents 95% of the samples. The curve is a plot of the Cumulative Turn Around Time (the fraction of samples with a Turn Around Time less than the x-axis value) showing that 95% of the samples will have a Turn Around Times of 43 minutes or less, and 100% of the samples will have Turn Around Times of 53 minutes or less, thus allowing visualization of laboratory performance metrics.

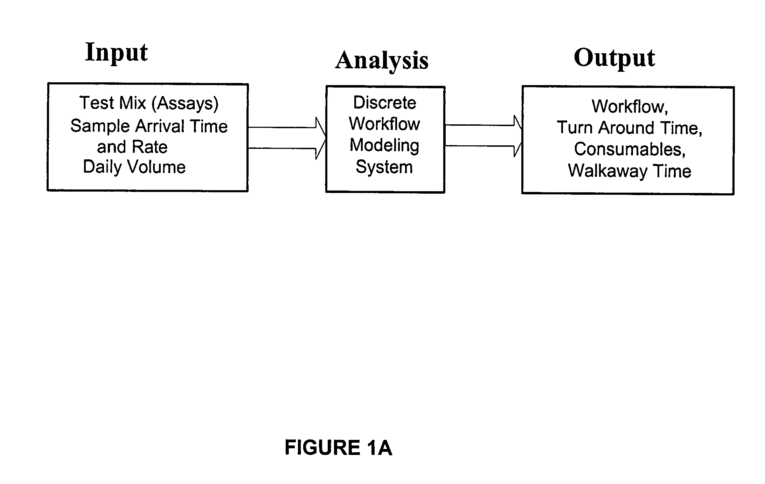

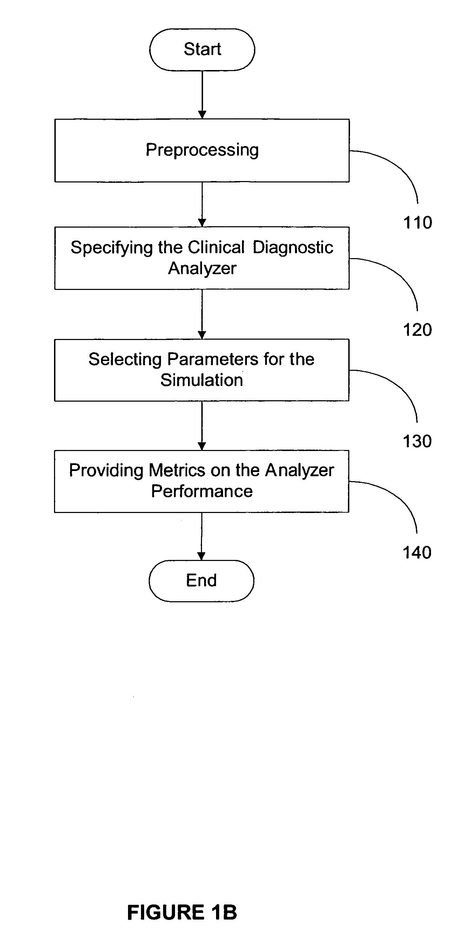

Figure 1A illustrates the broad outlines of the preferred embodiment for implementing the simulation. A preferred method for estimating processing of clinical samples at a site, illustrated in Figure 1B, preferably comprises preprocessing customer selected input data (step 110 of Figure 1B). Preprocessing of customer selected input data allows the preprocessing functionality to be tailored to the type of data provided by customers while reducing the need to change rest of the design to provide customizable evaluation service for different types of analyzers and data types.

The preferred method also comprises specifying a clinical diagnostic analyzer to be simulated (step 120 of Figure 1B); selecting at least one parameter (step 130 of Figure 1B) from the group consisting of a test mix, a retest rate, and delay due to retesting; simulating performance of the clinical diagnostic analyzer by executing a simulation software; and providing a measure of the performance of the clinical diagnostic analyzer (step 140 of Figure 1B) in a report including, for pseudo-samples marked Routine or STAT, at least one member of the group consisting of a Maximum Turn Around Time, an Average Turn Around Time, a 95% Turn Around Time, a Maximum Throughput, Consumable Usage, a Walk-Away Time, and a Downtime. Advantageously, the method includes a report on the performance metrics including in the form of an Arrival and Exit Rate Graph. A graph of Cumulative and % Turn Around Times may also be provided.

Preferably, in step 110 the customer selected data is converted into a standard format indicating at least a incoming time, the tests requested, the incubation time and other specifications. If the user does not specify a clinical diagnostic analyzer (in step 120), then a default clinical diagnostic analyzer specification is invoked. The parameters for the simulation may include a probabilistic prediction of whether a test result is out of range, and thus requires retesting and other probabilistic measures. These test the system against realistic failure rates.

Another preferred embodiment implements the simulation software by storing it on a memory device, which can be plugged into or coupled to a computing device with a processor and buffer memory for executing software instructions. The software instructions include commands for executing the simulation software package to evaluate the performance of a clinical diagnostic analyzer based on user approved data and user selected configuration of the clinical diagnostic analyzer, for instance as outlined broadly in Figures 1A-B. Some familiar memory devices are the hard drives in computers, particularly notebook computers, Universal Serial Bus based portable memory devices, floppy disks, Secure Digital Cards, DVDs, CDs and the like. These devices are useful for either being directly coupled to the computing device or for being used as a component to provide the instructions, for instance, by copying, directly or indirectly, the simulation software into a computing device. The simulation software, when executed, preferably causes generation of the report on the performance of the clinical diagnostic analyzer for STAT and Routine samples as well as cumulative measures that do not distinguish between Routine and Stat samples.

Some preferred metrics in the report include a Maximum Turn Around Time, an Average Turn Around Time, a 95% Turn Around Time, a Maximum Throughput, Consumable Usage, a Walk-Away Time, and a Downtime, wherein the report may further include an Arrival and Exit Rate Graph and/or a graph over time of the Cumulative and % Turn Around Times. Figure 5 includes an example of the latter.

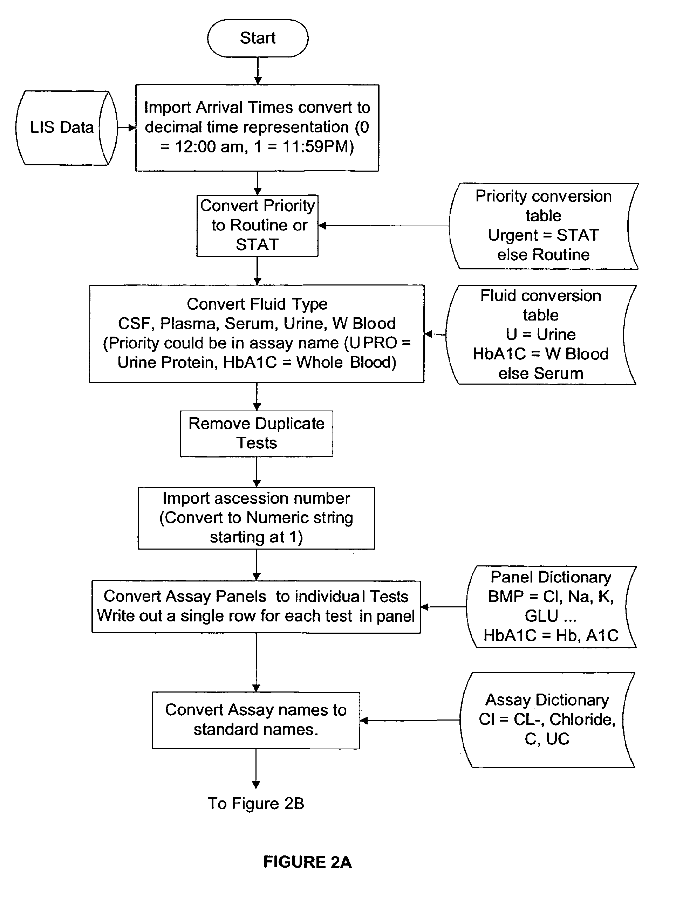

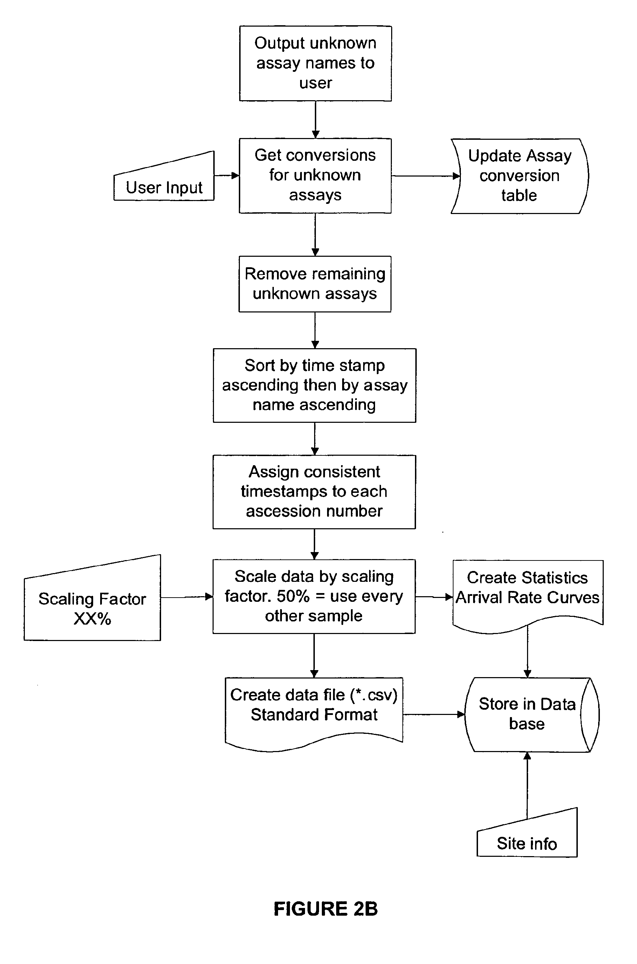

Another preferred embodiment is a simulation apparatus for simulating the performance of a clinical diagnostic analyzer. Such an apparatus may be modeled as shown in Figure 3. Figure 3 shows a preprocessor for formatting and organizing customer approved input data into a sample worklist. This data includes data corresponding to standard (Routine) and urgent (STAT) samples. Sample data are picked from the sample worklist to generate pseudo samples in accordance with a simulation clock. The pseudo samples are then processed for tests, such as those illustrated in the ASSAY TYPE TABLE, which is not an exhaustive listing but merely indicative of the large number of tests supported by the better clinical analyzers. A detailed description of the preprocessor is provided in Figures 2A-B, which organize the customer-approved data into a Sample Worklist, Analyzer Definitions and Assay Protocols data stores shown in Figure 3.

The simulation apparatus also includes a simulator consistent with the selected clinical diagnostic analyzer definition. The analyzer definition is essentially a description of the particular analyzer under consideration. This simulator operates as illustrated by Figure 3 to allow estimation of the time required for each step while preferably also accounting for the consumables expected to be required for processing the samples.

Preferably, the simulation apparatus includes a users' interface to provide an output. The output, preferably, includes one or more of the Maximum Turn Around Time, the Average Turn Around Time, the 95% Turn Around Time, the Maximum Throughput, Consumable Usage, the Walk-Away Time, and the Downtime. Further, the output may further include an Arrival and Exit Rate Graph, illustrative example of which are presented in Figures 4A-B.

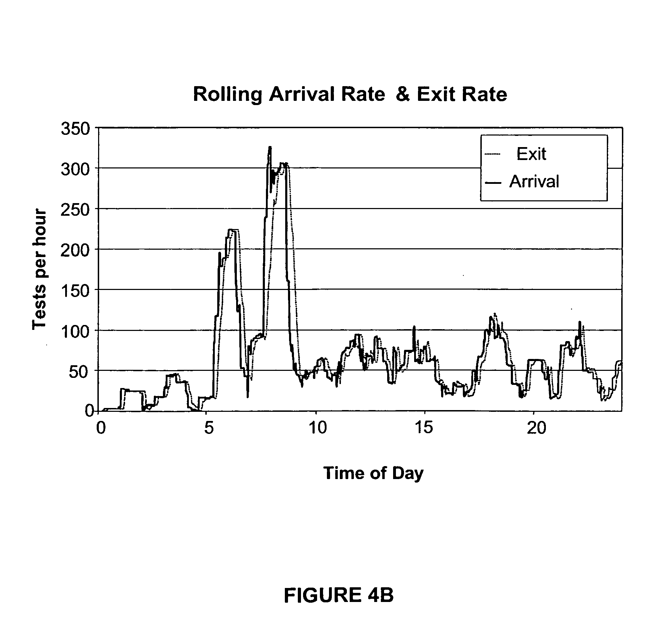

A preferred embodiment includes means for preprocessing customer selected input data, for instance, as shown in Figures 2A-B and 3; means for specifying a clinical diagnostic analyzer to be simulated, for instance, with data corresponding to a test mix, delay due to retesting, and downtime; means for simulating performance of the clinical diagnostic analyzer, for instance, as shown in Figure 3; and means for reporting metrics as the result of the simulation. Such reported metrics may include one or more of the Maximum Turn Around Time, the Average Turn Around Time, the 95% Turn Around Time, the Maximum Throughput, Consumable Usage, the Walk-Away Time, and the Downtime. Preferably these data are illustrated with the help of an Arrival and Exit Rate Graph, an example of which is shown in Figure 4.

A preferred apparatus for simulating a clinical diagnostic analyzer includes means for providing standardized data from data stored in a data store, the means for providing including any necessary preprocessing of the stored data as illustrated by Figures 2A-B; means for accessing at least one clinical diagnostic analyzer definition, which is illustrated in Figure 3; means for identifying assay protocols for processing pseudo-samples extracted from the standardized data; which is also illustrated in Figure 3; means for scheduling processing of a plurality of pseudo-samples from the pseudo-samples extracted from the standardized data as is illustrated in Figure 3; means for evaluating whether a test result on the plurality of pseudo-samples requires retesting as is illustrated in Figure 3 in the decision box 'Test Results out of range;' and means for collecting performance metrics on the plurality of pseudo-samples such as the database 'Test Process Log' and the document 'consumable Usage Statistics' shown in Figure 3. The performance metrics may include the Maximum Turn Around Time, the Average Turn Around Time, the 95% Turn Around Time, the Maximum Throughput, Consumable Usage, the Walk-Away Time, and the Downtime. The performance metrics may be presented with the aid of an Arrival and Exit Rate Graph.

Another preferred embodiment is a software package comprising means for preprocessing customer selected input data; means for specifying a clinical diagnostic analyzer to be simulated with data collected for at least one member of the group consisting of a test mix, delay due to retesting, and downtime; means for simulating performance of the clinical diagnostic analyzer; and means for reporting data collected in the simulation for at least one of standard samples, urgent samples and combined standard and urgent samples, wherein the means for reporting data include at least one member of the group consisting of a Maximum Turn Around Time, an Average Turn Around Time, a 95% Turn Around Time, a Maximum Throughput, Consumable Usage, a Walk-Away Time, and a Downtime, wherein the means for reporting data may further include an Arrival and Exit Rate Graph and a Cumulative Turn Around Time Graph.

Means for preprocessing customer selected input data generate standardized input from the customer selected data. If the customer selected data is already in a form acceptable to the means for simulating performance, then no conversion of the data into another form is required. Else, the selected data is transformed into a format suitable for the means for simulating performance.

Means for specifying a clinical diagnostic analyzer to be simulated allow selection of an analyzer from a list of analyzers for which simulation details are available. In a preferred embodiment, further, the properties of a newly specified analyzer may be input, for instance by specifying the supported operations, the time taken for the steps in each of the supported operations and the error and retesting rates.

Means for simulating performance compute the estimated time taken to perform various operations depending on the customer selected input data, which includes a specification of the tests to be performed. The time taken for each of the steps is conveniently estimated from the Scheduler in the clinical analyzer of interest and the expected frequency of retesting and errors. A Scheduler in a clinical diagnostic analyzer specifies the operations to be carried out at a particular time on a particular sample in order to perform a specified assay. It also receives confirmation that the specified operations were carried out at the proper time in its role as a controller. If the specified operation was not performed, or performed too late, the Scheduler may detect the error and flag the affected result. Thus, it has the appropriate information about the time taken to perform an assay.

In preferred embodiments the frequency of retesting and the error rates may be further specified to better evaluate the effect of such parameters on the performance of the clinical analyzer of interest. The means for simulating performance adds the times and resources required for various operations to arrive at statistics and performance metrics for the clinical analyzer of interest.

Means for reporting data collected in the simulation provide reports for standard samples and urgent samples. In a preferred embodiment, the urgent samples may be highlighted or otherwise distinguished, including when a combination of standard and urgent samples is presented to the clinical analyzer of interest. In a preferred embodiment, the output data from the means for reporting data include at least one member of the group consisting of a Maximum Turn Around Time, an Average Turn Around Time, a 95% Turn Around Time, a Maximum Throughput, Consumable Usage, a Walk-Away Time, and a Downtime, wherein the means for reporting data may further include an Arrival and Exit Rate Graph and a Cumulative Turn Around Time Graph. These parameters help describe the performance of the analyzer and provide an estimate of the cost of ownership of the clinical analyzer of interest.

The disclosed embodiments include an apparatus. Such an apparatus for simulating a clinical diagnostic analyzer comprises means for providing standardized data from data stored in a data store, the means for providing standardized data including any necessary preprocessing of the stored data; means for accessing a clinical diagnostic analyzer definition to provide a definition of at least one clinical diagnostic analyzer; means for identifying assay protocols for processing pseudo-samples extracted from the standardized data; means for scheduling processing to order steps for processing a plurality of pseudo-samples from the pseudo-samples extracted from the standardized data; means for evaluating retesting to detect whether a test result on the plurality of pseudo-samples requires retesting; means for collecting performance metrics on the plurality of pseudo-samples, wherein the performance metrics are selected from the group consisting of a Maximum Turn Around Time, an Average Turn Around Time, a 95% Turn Around Time, a Maximum Throughput, Consumable Usage, a Walk-Away Time, and a Downtime, wherein the means for collecting performance metrics may further include an Arrival and Exit Rate Graph and a Cumulative Turn Around Time Graph.

Means for providing standardized data convert the customer selected data into a standardized format. If the customer selected data is already in an acceptable form, then no conversion of the data into another form is required.

Means for accessing a clinical diagnostic analyzer definition provide access to either a default clinical diagnostic analyzer definition or to a particular clinical diagnostic analyzer definition. In most instances, it is expected that such a definition includes timing details underlying Scheduler operations. Such means may be in the form of an electronic memory or be implemented as part of a user interface designed to accept a clinical diagnostic analyzer definition, including by specifying a memory location or data structure with the required information.

Means for identifying assay protocols allow specification of the supported assays. Some exemplary assays are listed in the ASSAY TYPE TABLE. This illustrative listing is not limiting as additional or fewer than the listed assays may be supported on a particular analyzer, including based on local needs or business consideration. Such means may be in the form of an electronic memory or be implemented as part of a user interface designed to accept a specification of supported assays.

Means for scheduling processing allows estimation of the steps and the time and other resources required for processing of the samples.

Means for evaluating retesting make the simulation more realistic by allowing incorporation of expected error rates and the retesting due to errors to better evaluate the performance of the analyzer. Retesting decision making is also illustrated in Figure 3.

Means for collecting performance metrics collects and organizes the data on time and resources required for the simulated run to efficiently communicate the performance of the analyzer in the context of cost and management decision making. Some example metrics reportable by a preferred embodiment include one or more of a Maximum Turn Around Time, an Average Turn Around Time, a 95% Turn Around Time, a Maximum Throughput, Consumable Usage, a Walk-Away Time, and a Downtime, wherein the means for collecting performance metrics may further include an Arrival and Exit Rate Graph and a Cumulative Turn Around Time Graph. Figure 3 illustrates the time and consumable usage data collection for generating metrics of interest.

One skilled in the art will appreciate embodiments include estimating the performance of not just one or a few clinical diagnostic analyzers, but also a plurality of clinical diagnostic analyzers configured to operate together to process an incoming stream of samples. If several clinical diagnostic analyzers of the same type are deployed, then the input samples can just be divided evenly between them. However, if the clinical diagnostic analyzers are of different types, the individual characteristics of the analyzers need to be taken into account to evaluate performance of the entire configuration. One such exemplary embodiment is illustrated in Fig. 7.

Fig. 7 shows a simulation using three different kinds of clinical diagnostic analyzers. The first type of clinical diagnostic analyzer handles chemistry assays and immunoassays in an integrated setup with a scheduler to coordinate resource use to improve throughput. An example of such an analyzer is the VITROS 5600™ clinical diagnostic analyzer manufactured by Ortho Clinical Diagnostics®. VITROS 5600™'s Master Scheduler is implemented to support as many as three chemistry platforms: (i) microslides using dry chemistry, (ii) microtips for wet chemistry, and (iii) microwells using wet chemistry. VITROS 5600™ employs a robotic arm and pipettors shared between the various chemistry platforms to provide random access to input patient samples. The robotic arm allows aliquots to be drawn from a single input patient sample for each of the tests carried out on the analyzer, including tests carried out on different platforms integrated into the analyzer. This analyzer supports dry chemistry based tests on microslides for analytes such as Na, K, Cl⁻ and the like as well as immunoassays and other assays using its microwell and microtip platforms. The second type of clinical diagnostic analyzer, which is also a combinational clinical analyzer, includes fewer platforms. An example of such an analyzer is the VITROS 5,1 FS™ manufactured by Ortho Clinical Diagnostics®. The VITROS 5,1 FS™ supports microslide and microtip based assays only. The third type of clinical diagnostic analyzer has a more limited platform and may have as few as one platform. Examples include analyzers that only support immunoassays, such as VITROS ECi™ and VITROS 3600™ manufactured by Ortho Clinical Diagnostics®. Although clinical diagnostic analyzers manufactured by Ortho Clinical Diagnostics® are described here as preferred, the methods and teachings are applicable to other clinical diagnostic analyzers as well. Accordingly, one having ordinary skill in the art will readily modify the formulae and other criteria described in the examples herein for evaluating configurations based, wholly or in part, on analyzers manufactured by manufacturers other than Ortho Clinical Diagnostics®.

For the simulation, a strategy similar to Flow 700 outlined in Fig. 7 may be utilized. In Flow 700, LIS data is preprocessed to sort it based on the types of assays that are to be carried on each sample. Preferably, each type of clinical diagnostic analyzer uses an input patient sample to carry out one or more types of tests on small aliquots drawn from the sample. Thus, preferably a single sample is not split up between two or more different analyzers to reduce complexity, sample loss, and reduced throughput.

Input LIS data 705 is evaluated by Parser 710 to identify patient samples requiring tests based on each of microtips, microslides and microwells. These are assigned for processing by the versatile Analyzer I. If multiple Analyzer Is are deployed, then the samples are proportionally reduced (most output parameters can be estimated by simply scaling the performance of a single analyzer) by Reducer 725. Reducer 725 also tracks samples that cannot be processed by Analyzer II and Analyzer III due to their unavailability as determined in Decision Blocks 730 and 735 because Analyzer I is versatile enough to handle all types of tests. The total number of samples to be processed by Analyzer I are preferably reduced proportionally by Reducer 725 in the simulation for computational efficiency as describe above.

Input LIS data 705 is evaluated by Parser 715 to identify patient samples requiring tests based on each of microtips and microslides. These are provisionally assigned for processing by Analyzer 2. Decision Block 730 assigns such samples to Analyzer 1 if no Analyzer 2 is available. If Analyzer 2 is available, Decision Block 740 evaluates the First Condition.

The number of samples requiring both Chemistry and immunoassays > (the number of samples requiring Chemistry assays) / (Number of analyzers with Chemistry assays capability at Site)

If the First Condition is not satisfied, some of such samples are assigned to Analyzer 1 via the Reducer 745 and Summation Block 750, which also receives the output of Reducer 725. Reducer 745 evaluates the following for making its reduction to generate the input to Summation Block 750:

$$\frac{Total\_Samples\_with\_Chem\_Tests}{\#Analyzers\_with\_Chem\_Capability} - \frac{Samples\_with\_IA\_and\_Chem\_Tests}{\#Analyzer\_1s}$$

If the First Condition is not satisfied then Reducer 755 proportionally reduces the number of samples based on the number of Analyzer 2s to generate part of the sample input for Analyzer 2.

If the First Condition is satisfied, then, control flows to Reducer 760, which proportionally reduces the number of samples based on the number of Analyzer 2s to generate the remaining part of the sample input for Analyzer 2.

Input LIS data 705 is evaluated by Parser 720 to identify patient samples requiring immunoassays only. These are provisionally assigned for processing by Analyzer 3. Decision Block 735 assigns such samples to Analyzer 1 if no Analyzer 3 is available. If Analyzer 3 is available, Decision Block 765 evaluates the Second Condition.

The number of samples requiring both Chemistry and immunoassays > (the number of samples requiring immunoassays) / (Number of analyzers with immunoassay capability at Site)

If the Second Condition is not satisfied, some of such samples are assigned to Analyzer 1 via the Reducer 770 and Summation Block 750, which also receives the output of Reducer 725 and Reducer 745. Reducer 770 evaluates the following for making its reduction to generate the input to Summation Block 750:

$$\frac{Total\_Samples\_with\_IA\_Tests}{\#\,Analyzers\_with\_IA\_Capability} - \frac{Samples\_with\_IA\_and\_Chem\_Tests}{\#\,Analyzer\_1s}$$

If the Second Condition is not satisfied then Reducer 775 proportionally reduces the number of samples based on the number of Analyzer 3s to generate part of the sample input for Analyzer 3.

If the Second Condition is satisfied, then, control flows to Reducer 780, which proportionally reduces the number of samples based on the number of Analyzer 3s to generate the remaining part of the sample input for Analyzer 3.

The simulation then computes the performance of the entire configuration by computing the time required for handling the samples, the resources consumed and other output parameters described previously. The respective performance of each of the analyzer types may be computed by using their respective Schedulers as described previously. This approach does not result in a significant performance penalty or require very sophisticated resource hungry implementations for analyzing the performance of relatively complex configurations of clinical diagnostic analyzers.

One skilled in the art will appreciate that the above disclosure is susceptible to many variations and alternative implementations without departing from its teachings or spirit. The scope of the claims appended below includes such modifications. Further, each reference discussed and cited herein is hereby incorporated herein by reference in its entirety.

## Claims

1. A method for estimating performance of at least an analyzer for processing test samples at a site comprising the steps of:

    preprocessing customer selected input data;
    specifying a clinical diagnostic analyzer to be simulated;
    selecting at least one parameter from the group consisting of a test mix, a retest rate, and rescaling of data, wherein the rescaling reflects the number of analyzers being deployed;
    simulating performance of the clinical diagnostic analyzer by executing a simulation software; and

    providing a measure of the performance of the clinical diagnostic analyzer, for pseudo-samples marked Routine or STAT, at least one member of the group consisting of a Maximum Turn Around Time, an Average Turn Around Time, a 95% Turn Around Time, a Maximum Throughput, Consumable Usage, a Walk-Away Time, and a Downtime, wherein the performance measure may further include an Arrival and Exit Rate Graph and a Cumulative Turn Around Time Graph.

2. The method of claim 1, wherein the preprocessing step converts the customer selected data into a standard format.

3. The method of claim 1, wherein failure to manually specify the clinical diagnostic analyzer to be simulated invokes a default clinical diagnostic analyzer specification.

4. The method of claim 1, wherein the at least one clinical diagnostic analyzer is part of a configuration of clinical diagnostic analyzers.

5. The method of claim 4, wherein the configuration of clinical diagnostic analyzers includes a plurality of diagnostic clinical analyzers of different types, wherein at least one of the plurality of diagnostic clinical analyzers does not support immunoassays.

6. The method of claim 4, wherein the configuration of clinical diagnostic analyzers includes a plurality of diagnostic clinical analyzers of different types, wherein at least one of the plurality of diagnostic clinical analyzers does not support chemistry assays

7. A portable software instruction bearing memory device for providing software instructions to at least one computing device, the computing device comprising a processor and buffer memory; the software instructions including commands for executing a simulation software package to evaluate the performance of a candidate clinical diagnostic analyzer based on user approved data and user selected configuration of the candidate clinical diagnostic analyzer whereby the simulation software package causes generation of a report on the performance of the candidate clinical diagnostic analyzer for pseudo-samples marked Routine and STAT.

8. The software instruction bearing memory device of claim 7, wherein the software instruction bearing memory device is implemented using optical media, magnetic media, and portable memory media devices.

9. The software instruction bearing memory device of claim 7, wherein the software instruction bearing memory device is suitable for copying the instructions to a computer instruction bearing medium whereby causing execution of commands simulating the software package to evaluate the performance of the clinical diagnostic analyzer based on the user approved data and the user selected configuration of the clinical diagnostic analyzer whereby the simulation software package causes generation of the report on the performance of the candidate clinical diagnostic analyzer for samples marked standard and urgent.

10. The software instruction bearing memory device of claim 7, wherein the report includes at least one member of the group consisting of a Maximum Turn Around Time, an Average Turn Around Time, a 95% Turn Around Time, a Maximum Throughput, Consumable Usage, a Walk-Away Time, and a Downtime, wherein the report may further include an Arrival and Exit Rate Graph and a Cumulative Turn Around Time Graph.

11. The software instruction bearing memory device of claim 7, wherein the software instructions include rules for evaluating a configuration of clinical diagnostic analyzers.

12. The software instruction bearing memory device of claim 11, wherein the rules for evaluating the configuration of clinical diagnostic analyzers include configurations comprising diagnostic clinical analyzers of different types, wherein at least one diagnostic clinical analyzer does not support one member of the set consisting of immunoassays and chemistry assays.

13. A simulation apparatus for simulation performance of a clinical diagnostic analyzer comprising:

   a preprocessor for formatting and organizing customer approved input data including data corresponding to Routine and STAT samples;
   a simulator based on a selected clinical diagnostic analyzer definition; and
   a user interface providing an output, for samples marked standard and urgent, comprising at least one member of the group consisting of a Maximum Turn Around Time, an Average Turn Around Time, a 95% Turn Around Time, a Maximum Throughput, Consumable Usage, a Walk-Away Time, and a Downtime, wherein the output may further include an Arrival and Exit Rate Graph and a Cumulative Turn Around Time Graph.

14. The simulation apparatus of claim 13, wherein the simulator uses more than one clinical diagnostic analyzer definition for simulating the performance of a configuration of diagnostic clinical analyzers.

15. The simulation apparatus of claim 14, wherein rules for evaluating the configuration of clinical diagnostic analyzers include configurations comprising diagnostic clinical analyzers of different types, wherein at least one diagnostic clinical analyzer does not support one member of the set consisting of immunoassays and chemistry assays.

16. The simulation apparatus of claim 14, wherein customer approved input data is divided for processing by clinical diagnostic analyzers in the configuration of clinical diagnostic analyzers taking into account the tests to be simulated on samples included in the customer approved input data and the analyzer capabilities.

17. A software package comprising:

   means for preprocessing customer selected input data;
   means for specifying a clinical diagnostic analyzer to be simulated with data collected for at least one member of the group consisting of a test mix, delay due to retesting, and downtime;
   means for simulating performance of the clinical diagnostic analyzer; and
   means for reporting data collected in the simulation for at least one of standard samples, urgent samples and combined standard and urgent samples, wherein the means for reporting data include at least one member of the group consisting of a Maximum Turn Around Time, an Average Turn Around Time, a 95% Turn Around Time, a Maximum Throughput, Consumable Usage, a Walk-Away Time, and a Downtime, wherein the means for reporting data may further include an Arrival and Exit Rate Graph and a Cumulative Turn Around Time Graph.

**18.** Apparatus for simulating a clinical diagnostic analyzer comprising:

means for providing standardized data from data stored in a data store, the means for providing including any necessary preprocessing of the stored data;
means for accessing at least one clinical diagnostic analyzer definition;
means for identifying assay protocols for processing pseudo-samples extracted from the standardized data;
means for scheduling processing of a plurality of pseudo-samples from the pseudo-samples extracted from the standardized data;
means for evaluating whether a test result on the plurality of pseudo-samples requires retesting;
means for collecting performance metrics on the plurality of pseudo-samples, wherein the performance metrics are selected from the group consisting of a Maximum Turn Around Time, an Average Turn Around Time, a 95% Turn Around Time, a Maximum Throughput, Consumable Usage, a Walk-Away Time, and a Downtime, wherein the means for collecting performance metrics may further include an Arrival and Exit Rate Graph and a Cumulative Turn Around Time Graph.

FIGURE 1A

**FIGURE 1B**

**FIGURE 2A**

Output unknown assay names to user

User Input → Get conversions for unknown assays → Update Assay conversion table

Remove remaining unknown assays

Sort by time stamp ascending then by assay name ascending

Assign consistent timestamps to each ascession number

Scaling Factor XX% → Scale data by scaling factor. 50% = use every other sample → Create Statistics Arrival Rate Curves

Create data file (*.csv) Standard Format → Store in Data base

Site info

**FIGURE 2B**

**FIGURE 3**

## Rolling Arrival Rate and Exit Rate

FIGURE 4A

## Rolling Arrival Rate & Exit Rate

FIGURE 4B

FIGURE 5

EP 2 037 282 A2

| | | | | Site Data | | | | Routine Turn Around Time (minutes) All Assays | |
|---|---|---|---|---|---|---|---|---|---|
| Site & Country | Yearly Site Volume | Hours of Operation per Day | Chemistry Tests/Day Per System | Immunoassay Tests/Day Per System | % Immunoassay Tests/Day | % Tests in Busiest Hour | Number of Systems | Mean | 95% Cumulative |
| US | 2 million | 24 | 4036 | 356 | 8% | 18% | 2 | 15 | 36 |
| France | 1.6 million | 24 | 2715 | 297 | 10% | 30% | 2 | 34 | 79 |
| US | 1 million | 24 | 2196 | 523 | 19% | 15% | 2 | 18 | 47 |
| Germany | 780,000 | 12 | 1596 | 728 | 31% | 19% | 2 | 31 | 67 |
| Italy | 550,000 | 8 | 1035 | 174 | 14% | 37% | 2 | 23 | 42 |
| US | 220,000 | 24 | 1195 | 202 | 14% | 16% | 1 | 12 | 23 |

Table Illustrating Variability in Operations from Site to Site

FIGURE 6

**EP 2 037 282 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7076474 B
- US 20060031112 A
- US 20070078692 A
- US 6768968 B